# EUROPEAN PATENT APPLICATION

(11) **EP 4 273 234 A1**
(43) Date of publication of application: **08.11.2023**
(21) Application number: 21915348.3
(22) Date of filing: 29.12.2021
(51) Int. Cl.: C12N 5/10, A61K 35/17, A61K 35/545, A61P 35/00, C12N 5/074, C12N 5/0783, C12N 15/09, C12N 15/12, C12N 15/85, C12N 15/86

(54) **METHOD FOR PRODUCING REGENERATED T CELL VIA IPS CELL**

(30) Priority: 04.01.2021 JP 2021000176; 19.04.2021 JP 2021070534
(71) Applicant: Thyas Co. Ltd., Kyoto-shi, Kyoto 606-8304 (JP); KOTAI Biotechnologies, Inc., Suita-shi Osaka 565-0871 (JP)
(72) Inventor: KANEKO, Shin, Kyoto-shi, Kyoto 606-8304 (JP); GONOTSUBO, Ryosuke, Kyoto-shi, Kyoto 606-8304 (JP); OSAWA, Mitsujiro, Kyoto-shi, Kyoto 606-8304 (JP); HITOSHI, Yasumichi, Kyoto-shi, Kyoto 606-8304 (JP); YAMASHITA, Kazuo, Suita-shi, Osaka 565-0871 (JP); SAX, Nicolas Claude Paul, Suita-shi, Osaka 565-0871 (JP)
(74) Representative: De Clercq & Partners
(86) International application number: PCT/JP2021/049028
(87) International publication number: WO 2022/145490

(57) **Abstract**

[Problems]

To provide a method for producing regenerated T cells via iPS cells into which TCRs isolated from tumor tissue-infiltrating CD106-positive T cells have been introduced.

[Solutions]

Provided is a method for producing regenerated T cells via iPS cells, comprising: 1. A step for preparing cDNAs respectively encoding a TCR α chain and a TCR β chain from individual cells in a CD106-positive T cell population obtained from subjects and having reactivity with a tumor-related antigen; 2. A step for reprogramming peripheral blood mononuclear cells which are obtained from the subjects and from which B cells and T cells have been removed or T cells into iPS cells, and selecting iPS cell clones having high efficiency of differentiation into T cells from the obtained iPS cells; 3. A step for introducing the cDNAs into the iPS cell clones, hematopoietic stem cells differentiated from the iPS cell clones, immature T cells differentiated from the hematopoietic stem cells, or mature T cells differentiated from the immature T cells; and 4. A step for performing the differentiation of the iPS cell clones having the cDNAs introduced therein, the hematopoietic stem cells or the immature T cells which have been obtained in step 3 into mature T cells and the proliferation of the mature T cells.

## Description

### TECHNICAL FIELD

The present invention relates to a method for producing antigen-specific regenerated T cells by introducing a T cell receptor obtained from CD106-positive T cell population to iPS cells derived from non-T non-B cells, monocytes, or T cells, regenerated T cells produced by the method, a preventive or therapeutic agent for cancer comprising the regenerated T cells as an active ingredient, a pharmaceutical composition comprising the regenerated T cells, and a method for preventing or treating cancer using the pharmaceutical composition.

### BACKGROUND TECHNOLOGY

T cells play a central role in immune response to foreign pathogens such as bacteria or viruses or abnormal cells such as cancer cells. Therefore, decrease in the function of T cells is considered to contribute to pathogen infection and onset of cancer. T cell replacement therapy or regenerative therapy to a patient with a disease caused by decrease in the function of T cells can be an extremely effective means for amelioration of conditions and treatment of the disease in the patient.

In research using humans and mice, it is known that in the case of performing T cell replacement therapy for infectious diseases or cancer, high therapeutic effects can be achieved by using the T cells that specifically recognize antigens possessed by foreign pathogens such as bacteria or viruses or abnormal cells such as cancer cells. On the other hand, the difficulty in securing enough amount of T cells, and exhaustion of T cells such as decrease in proliferation ability in T cells and decrease in immune response to antigens such as target cells have become obstacles in T cell replacement therapy. In addition, how to acquire tumor-specific T cells is a major challenge.

In order to overcome the above obstacles in T cell replacement therapy, a T cell replacement therapy has been proposed, wherein pluripotent stem cells (iPS cells) are established from antigen-specific T cells, proliferated, and then the regenerated T cells differentiated into T cells are used. Since the T cell receptors (TCRs) for recognizing target antigens are formed by gene reconstitution of genome, the TCR genes of the T cells before the reprogram are preserved in the iPS cells in which the T cells have been reprogramed. Therefore, by using target antigen-specific T cells as raw materials for producing iPS cells, it is possible to produce regenerated T cells which exhibit the same antigen specificity as the original T cells (patent document 1 and non-patent document 1).

For safe and efficient T cell replacement therapy, stringent antigen specificity is essential. However, it is reported (patent document 2) that the frequency of appearance of the T cells having the same gene reconstitution pattern as the original T cells is not necessarily high in the regenerated T cells obtained via the iPS cells from the T cells as described above. In addition, it is also reported (non-patent document 2) that CD8αβ regenerated T cells obtained via iPS cells from human T cells lose their antigen specificity by additional reconstitution of the TCR α chain gene in the CD8/CD4 double-positive stage.

### PRIOR ART LITERATURES

### Patent Documents

1. WO2011/ 096482 (a pamphlet)
2. WO2013/176197 (a pamphlet)

### Non-patent Documents

1. Nishimura T, et al. Generation of rejuvenated antigen-specific T cells by reprogramming to pluripotency and redifferentiation. Cell Stem Cell. 2013; 12:114-126.
2. Minagawa A, et al. Enhancing T cell receptor stability in rejuvenated iPSC-derived T cells improves their use in cancer immunotherapy. Cell Stem Cell. 2018; 23: 850-858.

### SUMMARY OF THE INVENTION

### Problems to be Solved by the Invention

In cancer treatment using regenerated T cells produced via iPS cells (iPS-T cells), it is important to use iPS-T cells that specifically recognize target antigens present in tumor cells or tumor tissues in order to ensure safety and effectiveness of iPS-T cell replacement therapy in cancer treatment. In addition, when an onset or a recurrence of cancer is detected, it is important to promptly initiate iPS-T cell replacement therapy in order to improve treatment outcomes.

An object of the present invention is to provide a method for rapidly producing iPS-T cells into which a TCR isolated from a tumor tissue-infiltrating CD106-positive T cell has been introduced, and iPS-T cells produced according to the method. Furthermore, another object of the present invention is to provide iPS-T cells produced according to the method, a pharmaceutical composition comprising the iPS-T cells, and a method for preventing or treating cancer using the pharmaceutical composition.

It has been found in recent studies that even for one antigen epitope, a plurality of T cell clones (5 to 10 clones) recognizes an antigen by using different TCR, respectively. At present, it is extremely difficult to maintain all antigen-specific T cell clones during the process of obtaining iPS-T cells from cells obtained from patients. Also, the possibility that clones differentiated to iPS-T cells are the T cell clones having an optimal TCR to damage the target are affected by individual difference of a subject from whom the cells for producing iPS-T cells are collected or production batch of iPS-T cells. In addition, in order to isolate T cells that specifically recognize tumors, it is necessary to administer tumor antigens to patients or to co-culture T cells and tumor antigens *in vitro.* However, when the tumor antigen is unknown, it is extremely difficult to employ such methods.

In addition, a long period of time is required currently for producing iPS-T cells using iPS cell clones derived from cells obtained from a patient. In order to promptly provide iPS-T cell replacement therapy to patients in need thereof, shortening the production period is a major challenge.

### Means for Solving the Problems

The following has been found by the present inventors. A CD106-positive T cell population isolated from tumor tissue is a T cell population that recognizes tumor-related antigens and has specific aggression against cancer cells. Different TCR pools with antigen specificity can be acquired from these T cell populations that recognize tumor-related antigens. iPS-T cells can be produced by introducing a TCR into iPS cells established from non-T non-B cells or monocytes or T cells. By selecting in advance the iPS cell clones with good differentiation efficiency into T cells, the quality of regenerated T cells can be homogenized, and production period can be shortened. At the same time, by adjusting the timing of acquisition of the TCR pool and establishment of iPS cells, the iPS-T cells can be produced rapidly. The TCR pool and the non-T non-B cells, monocytes, or T cells may originate from the same individual or from separate individuals. Consequently, the present invention was completed.

That is, the objects of the present invention are achieved by following inventions.
[1] A method for producing regenerated T cells via iPS cells, comprising:
   (1) a step for preparing cDNAs respectively encoding a TCR α chain and a TCR β chain from individual cells in a CD106-positive T cell population obtained from subjects and having reactivity with a tumor-related antigen;
   (2) a step for reprogramming peripheral blood mononuclear cells which are obtained from the subjects and from which B cells and T cells have been removed or T cells into iPS cells, and selecting iPS cell clones having high efficiency of differentiation into T cells from the obtained iPS cells;
   (3) a step for introducing the cDNAs into the iPS cell clones, hematopoietic stem cells differentiated from the iPS cell clones, immature T cells differentiated from the hematopoietic stem cells, or mature T cells differentiated from the immature T cells; and
   (4) a step for performing the differentiation of the iPS cell clones having the cDNAs introduced therein, the hematopoietic stem cells or the immature T cells which have been obtained in step (3) into mature T cells and the proliferation of the mature T cells.
[2] The method according to [1], wherein step (2) is carried out prior to or in parallel with step (1).
[3] The method according to [1] or [2], wherein the subjects in steps (1) and (2) are the same individuals and are those to be prevented or treated of cancer.
[4] The method according to [1] or [2], wherein the subjects in steps (1) and (2) are separate individuals from each other and the subjects in step (2) are those to be prevented or treated of cancer.
[5] The method according to [1] or [2], wherein the subjects in steps (1) and (2) are separate individuals from each other and the subjects in step (1) are those to be prevented or treated of cancer.
[6] The method according to [1] or [2], wherein the subjects in steps (1) and (2) are the same individuals or separate individuals from each other, and the subjects different from the subjects in steps (1) and (2) are those to be prevented or treated of cancer.
[7] The method according to [1] or [2], further comprising a step for selecting, from the T cells into which the cDNAs obtained in step (4) have been introduced, the T cells which do not exhibit an alloreaction to the cells derived from subjects to be prevented or treated of cancer.
[8] The method according to [1] or [2], further comprising a step for selecting, from the cDNAs prepared in step (1), the cDNAs encoding a TCR that do not induce an alloreaction to the cells derived from subjects to be prevented or treated of cancer.
[9] The method according to [7] or [8], wherein the cells derived from subjects to be prevented or treated of cancer are peripheral blood mononuclear cells.
[10] The method according to any one of [1] to [9], wherein the introduction of the cDNAs into the iPS cell clones, the hematopoietic stem cells differentiated from the iPS cell clones, the immature T cells differentiated from the hematopoietic stem cells, or mature T cells differentiated from the immature T cells in step (3) uses a virus vector, a non-viral vector, or a genome editing technique.
[11] The method according to [10], wherein the genome editing technique is CRISPR/Cas9 or TALEN.
[12] The method according to [10], wherein the non-viral vector is a transposon vector.
[13] The method according to [12], wherein the transposon vector is piggyBac ^{®} transposon vector.
[14] The method according to any one of [1] to [13], wherein the step (4) for performing the differentiation of the iPS cell clones having the cDNAs introduced therein, the hematopoietic stem cells or the immature T cells into mature T cells and the proliferation of the mature T cells is carried out in the presence of feeder cells and PHA (phytohemagglutinin), RetroNectin^{®} and anti-CD3 antibodies, or anti-CD3 antibodies and anti-CD28 antibodies.
[15] The method according to [14], wherein the feeder cells are autologous or allogeneic peripheral blood mononuclear cells.
[16] The method according to any one of [1] to [15], wherein the subjects in step (1) or (2) are patients of hepatoma, hepatoblastoma, gastric cancer, esophageal cancer, lung cancer, pancreatic cancer, renal cell cancer, breast cancer, ovarian cancer, skin cancer, such as malignant melanoma, bladder cancer, head and neck cancer, uterine cancer, cervical cancer, glioblastoma, prostate cancer, neuroblast, chronic lymphocytic leukemia, thyroid papilla cancer, colon cancer, brain tumor, sarcoma, or B-cell non-Hodgkin's lymphoma.
[17] The method according to any one of [1] to [15], wherein the subjects in step (1) or (2) are highly immunogenic cancer patients.
[18] The method according to [17], wherein the highly immunogenic cancer is hepatoma, hepatoblastoma, colon cancer, lung cancer, or malignant melanoma.
[19] The method according to any one of [1] to [18], wherein the T cell population in step (1) is CD3/CD106 double-positive.
[20] The method according to any one of [1] to [19], wherein the hematopoietic stem cells are CD34/CD43 double-positive.
[21] The method according to any one of [1] to [20], wherein the immature T cells are CD8 α chain/β chain double-positive.
[22] The method according to any one of [1] to [21], wherein the iPS cell clones with high differentiation efficiency selected in step (2), the hematopoietic stem cells differentiated from the iPS cell clones in step (3), the immature T cells differentiated from the hematopoietic stem cells, or the mature T cells differentiated from the immature T cells are preserved to construct a master cell bank.
[23] The method according to [22], wherein the preservation is cryopreservation.
[24] The method according to [22], wherein steps (3) and (4) are performed on the iPS cell clones, the hematopoietic stem cells, the immature T cells, or the mature T cells preserved in the master cell bank.
[25] The master cell bank according to [22], comprising the iPS cell clones, the hematopoietic stem cells, the immature T cells, or the mature T cells.
[26] Regenerated T cells produced by the method according to any one of [1] to [24].
[27] A pharmaceutical composition, containing the regenerated T cells according to [26].
[28] A method for preventing or treating cancer using the pharmaceutical composition according to [27].

### EFFECTS OF THE INVENTION

In the method of the present invention for producing regenerated T cells via iPS cells (iPS-T cells), after peripheral blood mononuclear cells or T cells from which T cells and B cells have been removed (non-T non-B cells or monocytes) are reprogramed, and iPS cells are obtained, iPS cell clones demonstrating good differentiation efficiency into T cells are selected in advance. Therefore, it is possible to ensure enough amount of iPS-T cells necessary for treatment in accordance with the timing required for the treatment. In addition, in the method of the present invention, in order to introduce TCR into iPS cell clones derived from non-T non-B cells or monocytes or T cells; hematopoietic stem cells differentiated from the iPS cell clones; immature T cells differentiated from the hematopoietic stem cells, or mature T cells differentiated from the immature T cells, in case of expanded culture of these cells after their differentiation into iPS-T cells, reconstitution of TCR gene is less likely to occur, and the antigen specificity of the introduced TCR is maintained. Furthermore, it is possible to produce iPS-T cells with few exhaustions caused by expanded culture. Moreover, in the production of iPS-T cells, since iPS cell clones demonstrating good differentiation efficiency into T cells are selected and used, it is possible to minimize the effects of variations in yield and degree of differentiation between the production batches of iPS-T cells and individual differences of subjects from whom cells are collected on the quality of obtained iPS-T cells, etc. Besides, it becomes possible to efficiently produce the T cells having TCRs that recognize antigens and efficiently damage targets.

According to the method of the present invention, since the cDNAs encoding TCR are prepared from individual cells in T cell population having genetic diversity, a cDNA group having various antigen specificity can be prepared. Since CD106-positive T-cell populations isolated from tumor tissue is a T cell population that recognizes tumor antigens, there is no need for prior immunization with tumor antigens or *in vitro* co-culture of T cells with tumor antigens in isolating a tumor-specific TCR. Therefore, according to the method of the present invention, it is possible to produce a tumor-specific iPS-T cell even when the production period of an iPS-T cell is shortened and the tumor antigen in a cancer patient is unknown. In addition, since the preparation and preservation of the iPS cell clones derived from non-T non-B cells or monocytes or T cells are carried out prior to or in parallel with the preparation of cDNAs encoding antigen-specific TCRs, iPS-T cells can be produced in a shorter period of time as compared with the case using a method wherein these are carried out sequentially. Therefore, in cancer treatment to which an iPS-T cell replacement therapy is applied, it becomes possible to quickly prepare iPS-T cells specific to an expressed antigen in cases of antigen change of tumor, resistance to treatment, or recurrence of cancer in a patient to be treated of cancer.

According to the method of the present invention, a subject from whom the T cells used for preparing cDNAs encoding TCRs are collected may be the same individual as or different individual from a subject who is a patient to be treated of cancer by iPS-T cell replacement therapy. Therefore, it becomes possible to prepare TCR pools corresponding to various antigens, and select the TCRs having optimal antigen specificity for individual cancer patient.

### BRIEF DESCRIPTION OF THE DRAWINGS

FIG. 1 shows an outline of a step for producing regenerated T cells from iPS cells in which non-T non-B cells or monocytes of peripheral blood have been reprogramed.
FIG. 2 shows details of a step for producing regenerated T cells from iPS cells in which non-T non-B cells or monocytes of peripheral blood have been reprogramed. In FIG. 2, the upper diagram shows a step for isolating the TCR genes from CD106-positive T cells and a step for verifying the antigen-specific reactivity, and the lower diagram shows a step for producing iPS cells transfected with TCR and a step for producing regenerated T cells from the iPS cells.
FIG. 3 shows a step for transfecting a TCR gene using a picggyBac^{®} transposon vector.
FIG. 4 shows a phenotype of regenerated T cells derived from iPS cells (tumor-related antigen-specific CD8-positive cytotoxic T cells).
FIG. 5 shows the results of analyzing telomere as a cell aging marker in regenerated T cells.
FIG. 6 shows the results of analyzing the expressions of PD-1 and TIGHT molecules as cell exhaustion markers in regenerated T cells.
FIG. 7 shows comparison of the amount of IL-2 and IFN-γ produced by stimulation with PMA and ionomycin in cytotoxic T cells (CD8 α chain/β chain double-positive cells) and regenerated T cells in peripheral blood mononuclear cells of a healthy subject.
FIG. 8 shows the expression of various markers in tumor infiltrating T cells. In each panel, cells in which the expression of the marker described in the panel is positive are shown colored. The cells are clustered by expression patterns, and the encircled portions correspond to tumor-cytotoxic T cells.
FIG. 9 shows IFN-γ production when tumor infiltrating T cells were autologous tumor stimulated in a subpopulation sorted by marker expression. It can be understood that IFN-γ is produced by autologous tumor stimulation only in CD106-positive cell sub-populations, and IFN-γ is produced by autologous tumor stimulation in marker-positive cell sub-populations other than CD106, but since IFN-γ is also produced in the marker-negative cell sub-population, the specificity is inferior to CD106.
FIG. 10 shows a phenotype of mature iPS cell-derived mature T cells in which a GPC3 antigen-specific TCR gene has been transfected. The CD19 gene is a gene incorporated in tandem with the TCR gene into the same picggyBac^{®} transposon vector and is used as a marker for gene insertion into a host chromosome and gene expression.
FIG. 11 illustrates a method for selecting the iPS cell clones that demonstrate high differentiation efficiency into T cells in the step for producing regenerated T cells from iPS cells in which peripheral blood T cells have been reprogramed.
FIG. 12 illustrates a method for performing genome editing on iPS cell clones selected as the cells with high differentiation efficiency into T cells and producing regenerated T cells in the step for producing regenerated T cells from iPS cells in which peripheral blood T cells have been reprogramed.
FIG. 13 illustrates a method for producing regenerated T cells which recognize cancer antigens from host T cells.
FIG. 14 shows an outline of a step for producing regenerated T cells from allogeneic iPS cells in which peripheral blood T cells have been reprogramed.

### EMBODIMENTS FOR CARRYING OUT THE INVENTION

### [T cells]

In the present invention, the "T cells" obtained from subjects is the cells expressing antigen receptors called T cell receptors (TCRs) on cell surface. The TCRs include heterodimers consisting of α and β chains and heterodimers consisting of γ and δ chains. T cells having TCR consisting of α and β chains are called αβ-type T cells, and T cells having TCR consisting of γ and δ chains are called γδ-type T cells. In one aspect of the present invention, the T cells of the present invention are preferably CD3/αβ-type T cells, but may be CD3/γδ-type T cells. The TCRs of the T cells in the present invention are transfected.

In the present invention, the "T cell population" refers to the cells as described above that are generally diverse in the gene sequences of antigen-recognizing T cell receptors (TCRs). Therefore, T cells collected from a living body are T cell populations having specificity to various antigens. The T cells existing *in vivo* have TCR gene sequences that differ from individual T cells due to occurrence of random recombination of TCR genes when T precursor cells develop and differentiate in the thymus, and may cause an immune response to any antigen. In the present invention, CD106 is a highly specific marker for identifying T cells having aggression to cancer cells, and the TCR of individual CD106-positive T cells is tumor antigen-specific. Thus, by considering the CD106-positive T cells as a group, it can be understood that the T cell population targets various tumor antigens for immunization. Therefore, CD106-positive T cell populations collected from a living body are those having genetic diversity in this sense.

In the present invention, a "tumor-related antigen" is an antigen that is expressed in a tumor-specific or non-specific manner, such as an antigen derived from a protein overexpressed in tumor cells and a mutant thereof, an antigen derived from a tumor virus, a certain type of differentiated antigen, or a new tumor-related antigen (neoantigens) due to a gene mutation, and a splice abnormality, etc. T cells responsive to a "tumor-related antigen" are recognized as CD106-positive T cells. Tumor-related antigens may be described herein as tumor antigens. In the case of a protein antigen, this may be a fragmented peptide (a peptide fragment). Examples of the antigens that are expressed in a tumor-specific or non-specific manner include WT1, GPC3, XAGE1, MUC1, MUC5AC, MUC6, EGFRvIII, HER-2/neu, MAGE A3, MAGEA1, telomerase, PRAME, SSX2/4, PSCA, CTLA-4, gp100, GD2, GD3, fucosyl GM1, GM3, sLe (a), glycolipid F77, mesothelin, PD-L1, trp1, trp2, CD19, CD20, CD22, ROR1, CD33, c-Met, p53, p53 mutant, NY-ESO-1, PSMA, ETV6-AML, CEA, PSA, AFP, hTERT, EpCAM, ALK, androgen receptor, EphA2, CYP1B1, OY-TES-1, MAD-CT-2, MelanA/MART1, survivin, Ras, Ras mutant, ERG, bcr-abl, XBP1, and the like, however they are not limited thereto. Examples of the viral antigens include inactivated viruses such as inactivated HBV and HPV, and proteins derived from various viruses such as EBV LMP1, EBV LMP2, EBNA (EBV nuclear antigen), HPV E1, HPV E2, HPV E6, HPV E7, HBV HBs, HTLV-1 Tax, and HBZ (HTLV-1 bZIP Factor), etc., however, they are not limited thereto.

In the present invention, "having reactivity to a tumor-related antigen" means that T cells react via TCR by specific binding/conjugation to epitope peptides derived from tumor-related antigens presented to major histocompatibility complex (MHC) class I or class II on antigen presenting cells, and refers to that the T cells do not react by binding/conjugation to other epitope peptides described above. Examples of the reactions via TCR of the T cells by binding/conjugation to epitope peptides derived from tumor-related antigens presented to MHC class I or class II include cytotoxicity, production of IFN-γ and granzyme, expression of T cell activation marker, and activation of transcription factors such as NF-AT.

In the present invention, T cells are preferably αβ T cells. Preferable collection source of T cells is peripheral blood because of its low invasiveness, but is not limited thereto. Examples of other preferable collection sources include all collection sources in the body, such as cancer tissue or tumor tissue, lymph node or other tissue or organ, or blood, umbilical cord blood, lymph fluid, interstitial fluid (inter-tissue liquid, inter-cell liquid and interstitial fluid), body cavity fluid (ascitic fluid, pleural fluid, pericardial fluid, cerebrospinal fluid, synovial fluid, and aqueous humor). In one aspect of the present invention, preferable T cells are those derived from tumor tissue. T cells derived from tumor tissue are usually tumor infiltrating T cells.

In the case where the subject is a cancer patient, the cancer of the cancer patient is selected from hepatoma, hepatoblastoma, gastric cancer, esophageal cancer, lung cancer, pancreatic cancer, renal cell cancer, breast cancer, ovarian cancer, skin cancer, such as malignant melanoma, bladder cancer, head and neck cancer, uterine cancer, cervical cancer, glioblastoma, prostate cancer, neuroblast, chronic lymphocytic leukemia, thyroid papilla cancer, colon cancer, brain tumor, sarcoma, or B-cell non-Hodgkin's lymphoma. The cancer is preferably hepatoma or hepatoblastoma.

### [iPS cell clones]

In the present invention, iPS cells are preferably prepared by reprograming non-T non-B cells, monocytes, or T cells. However, they are not limited thereto. The "non-T non-B cells" mean mononuclear cells that are classified neither as T cells nor as B cells. In the present invention, non-T non-B cells or monocytes can be prepared by collecting peripheral blood mononuclear cells from subjects and then removing the T cells and B cells contained in the mononuclear cells. Peripheral blood mononuclear cells can be isolated from human whole blood by a mononuclear cell separation solution. Lymphoprep^{®}can be exemplified as a mononuclear cell separation solution. In order to remove B cells and T cells from mononuclear cells, magnetic beads such as flow cytometry or MACS^{®} beads may be used by utilizing antibodies against CD19, CD20, CD22 which are surface antigens of B cells, or B cell receptors, and CD3, CD4 or CD8 which are surface antigens of T cells. T cells can be isolated from human whole blood by a mononuclear cell separation solution. Lymphoprep^{®}can be exemplified as a mononuclear cell separation solution. Purification of T cells can be performed by utilizing CD3, CD4 or CD8 which are surface antigens of T cells, or T cell receptors. For example, magnetic beads such as flow cytometry or MACS^{®} beads may be used.

Methods for producing iPS cells are known in the art. In the present invention, iPS cells can be preferably induced by introducing cell reprogramming factors into non-T non-B cells, monocytes, or T cells. Examples of the cell reprogramming factors include genes such as Oct3/4, Sox2, Sox1, Sox3, Sox15, Sox17, klf4, klf2, c-Myc, N-Myc, L-Myc, Nanog, Lin28, Fbx15, ERas, ECAT15-2, Tel1, β-catenin, Lin28b, Sall1, Sall4, Esrrb, Nr5a2, Tbx3, and Glis1, etc., or gene products. These cell reprogramming factors may be used alone or in combination. From the viewpoint of efficiently establishing iPS cells, among these cell reprogramming factors, it is preferable to introduce Oct3/4, Sox2, Klf4, and c-Myc (so-called Yamanaka's four factors) into the non-T non-B cells, monocytes, or T cells.

Methods for introducing cell reprogramming factors into the non-T non-B cells, monocytes, or T cells are not particularly limited, and those known in the art can be employed. For example, in a case of introducing a gene encoding the cell reprogramming factor into the non-T non-B cells or monocytes, a gene encoding the cell reprogramming factor (e.g., cDNA) can be inserted into an expression vector comprising a promoter functioning in non-T non-B cells, monocytes, or T cells, and the expression vector can be introduced into non-T non-B cells, monocytes, or T cells by infection or methods of lipofection, liposome and calcium phosphate coprecipitation, DEAE dextran, microinjection, or electroporation. In the case where the cell reprogramming factor is in the form of a protein and the protein is introduced into the non-T non-B cells, monocytes, or T cells, a method using a protein introduction reagent, a method using protein introduction domain fusion protein, electroporation method, and microinjection method can be exemplified. In the case where the cell reprogramming factor is in the form of a messenger RNA (mRNA) and the mRNA is introduced into the non-T non-B cells or monocytes, a method using an mRNA introduction reagent and a method of addition to a culture medium can be exemplified.

Examples of expression vectors used for transfection by infection include virus vectors such as lentivirus, retrovirus, adenovirus, adeno-associated virus, herpes virus, and Sendai virus and animal cell expression plasmids. However, from the viewpoints of less possibility of insertional mutagenesis, high transfection efficiency, and large number of copies of transfected genes, Sendai virus is preferably used to introduce a gene encoding the cell reprogramming factor into the non-T non-B cells, monocytes, or T cells.

Examples of the promoters used in the expression vector for transfecting a gene encoding the cell reprogramming factor into a non-T non-B cells, monocytes, or T cells include SRα promoter, SV40 promoter, LTR promoter, CMV promoter, RSV promoter, HSV-TK promoter, ubiquitin promoter, and the like. These promoters may be capable of controlling the expression of a gene inserted downstream of the promoter depending on the presence or absence of a drug such as tetracycline. The expression vector may comprise enhancer, poly A addition signal, selection marker gene (e.g., neomycin resistance gene), SV40 replication origin, and the like, in addition to promoter.

A culture medium used for culturing iPS cells obtained by reprogramming non-T non-B cells, monocytes, or T cells is not particularly limited. It can be prepared by using a culture medium for culturing animal cells as a basal culture medium, and adding thereto cytokines for maintaining undifferentiation potential of iPS cells. Examples of basal culture media include Iscove's Modified Dulbecco's Medium (IMDM), Medium 199, Eagle's Minimum Essential Medium (EMEM), αMEM medium, Dulbecco's modified Eagle's Medium (DMEM), Ham's F12 medium, RPMI 1640 medium, Fischer's Neurobasal Medium (Life Technologies), StemFit^{®} AK03N (Ajinomoto Healthy Supply), and a mixed medium thereof. A culture medium may be added with serum or may be serum-free. bFGF may be exemplified as a preferable cytokine, and the concentration thereof in a culture medium is, for example, 1 to 100 µg/mL (preferably 50 µg/mL).

In one aspect of the present invention, the method for culturing iPS cells may be adhesion culture or suspension culture, preferably adhesion culture. Examples of the methods for isolating iPS cells include physical methods using a cell scraper or the like, and methods using a dissociation solution with protease activity, or collagenase activity, or protease activity and collagenase activity (e.g., Accutase^{®} and Accumax^{®}, etc.).

In one aspect of the present invention, iPS cells are preferably subcultured in another incubator when they reach a cell density of 1×10³ to 1×10⁴ cells/cm², 1×10⁴ to 1×10⁵ cells/cm², or 1×10⁵ to 1×10⁶ cells/cm². The passage number may be any number as long as a required amount of iPS cells can be obtained, preferably 1 to 5 or 5 to 10.

The iPS cells produced consist of a large number of iPS cell clones. As will be described later, in order to select the iPS cell clones that demonstrate high differentiation efficiency into T cells, it is preferable to clone iPS cells by a colony pickup method. The colony pick-up method is not particularly limited. A method using Pipetman under a microscope, limiting dilution analysis, and a method using a fully automatic colony picker are used. The iPS cell clones obtained may be cultured expansively to construct a master cell bank. The "master cell bank" composed of the iPS cell clones is a single pool of single iPS cell clone that is dispensed and accumulated in an independent cell storage container. In the master cell bank, it is preferable to cryopreserve the iPS cell clones. Methods for cell cryopreservation are well known to those skilled in the art. For example, the cultured iPS cell clones can be harvested and washed with a buffer solution or a culture medium, enriched by centrifugation or the like after the cell number is counted, suspended in a medium for cryopreservation (e.g., a culture medium containing 10% DMSO), and then cryopreserved at a low temperature. The master cell bank of the present invention can be preserved in any facility or storage capable of cryopreservation. In case of cryopreservation, the storage temperature is not particularly limited as long as it is suitable for the preservation of cells. Examples thereof include -20°C, -80°C, and - 120°C to -196°C, preferably -150°C or lower.

"Differentiation efficiency" refers to the abundance ratio of hematopoietic stem cells, immature T cells and mature T cells, respectively in all viable cells in each differentiation stage from iPS cells into hematopoietic stem cells, from hematopoietic stem cells into immature T cells, and from immature T cells into mature T cells. The identification of differentiated cells in each differentiation stage can be performed by FACS analysis of surface markers. The differentiation efficiency into hematopoietic stem cells is indicated as the ratio of CD34/CD43 double-positive cells in all viable cells. The differentiation efficiency into immature T cells is indicated as the ratio of CD4/CD8 double-positive cells or that of CD5 positive cells in all viable cells. In addition, the differentiation efficiency into mature T cells is indicated as the ratio of the cells wherein CD8 α chain, CD8 β chain, TCR α chain, and TCR β chain are all positive in all viable cells.

"Differentiation efficiency is high." or "Differentiation efficiency is good/excellent." refer to that the ratio of CD34/CD43 double-positive cells, which are hematopoietic stem cells, is 5 to 15% or more in the differentiation from iPS cells into hematopoietic stem cells, the ratio of CD4/CD8 double-positive cells or that of CD5 positive cells, which are immature T cells, is 10% or more or 50% or more, respectively in the differentiation from hematopoietic stem cells into immature T cells, and the ratio of the cells wherein CD8 α chain, CD8 β chain, TCR α chain and TCR β chain are all positive is 50% or more in the differentiation from immature T cells into mature T cells.

The regenerated T cells of the present invention are preferably produced by firstly differentiating the iPS cell clones into hematopoietic stem cells, then differentiating the hematopoietic stem cells into immature T cells, and finally differentiating immature T cells into mature T cells that are CD8 single-positive T cells.

In the present invention, "hematopoietic stem cells" are cells capable of differentiating into hematopoietic cells such as lymphocytes, eosinophils, neutrophils, basophils, erythrocytes, and megakaryocytes, etc. Hematopoietic stem cells and hematopoietic progenitor cells (HPC) are not distinguished from each other, and are referred to as the same cells, unless otherwise specified. Hematopoietic stem cells/progenitor cells are recognized by that, for example, the surface antigens CD34 and CD43 are double-positive.

In the present invention, "immature T cells" refer to the T cells at each stage from T cells in which both TCR α and β chains are not expressed, through CD4/CD8 double-positive cells to CD8 single-positive cells, which express both TCR α and β chains. Immature T cells are preferably CD8 α chain/β chain double-positive.

In the present invention, "mature T cells" refer to the T cells that express TCR α and β chains and has progressed through CD4/CD8 double-positive cells to CD8 single-positive cells. Mature T cells are preferably CD8 α chain/β chain double-positive.

### [Culture of hematopoietic stem cells and immature T cells]

Hematopoietic stem cells are preferably produced by culturing iPS cells in a culture medium added with vitamin C. Here, "vitamin C" refers to L-ascorbic acid and a derivative thereof. A "derivative of L-ascorbic acid" means one that is converted into vitamin C by an enzymatic reaction *in vivo.* Examples of derivatives of L-ascorbic acid include vitamin C phosphate, ascorbyl glucoside, ethyl ascorbic acid, vitamin C esters, ascorbyl tetrahexyldecanoate, ascorbyl stearate, and ascorbic acid-2-phosphate-6-palmitate. A preferable derivative of L-ascorbic acid is vitamin C phosphate, and examples thereof include phosphoric acid-L-ascorbates such as phosphoric acid-sodium L-ascorbate or phosphoric acid-magnesium L-ascorbate. Vitamin C is contained in a culture medium at a concentration of, for example, 5 to 500 µg/mL.

A culture medium used for producing hematopoietic stem cells is not particularly limited. It can be prepared by using a culture medium for culturing animal cells as a basal culture medium, and adding vitamin C, etc. thereto. Examples of basal culture media include Iscove's Modified Dulbecco's Medium (IMDM), Medium 199, Eagle's Minimum Essential Medium (EMEM), αMEM medium, Dulbecco's modified Eagle's Medium (DMEM), Ham's F12 medium. RPMI 1640 medium, Fischer's medium, Neurobasal Medium (Life Technologies), StemPro34 (Life Technologies), and a mixed medium thereof. A culture medium may contain serum or may be serum-free. If necessary, a basal medium may contain one or more substances selected from, for example, albumin, insulin, transferrin, selenium, fatty acids, trace elements, 2-mercaptoethanol, thioglycerol, monothioglycerol, lipids, amino acids, L-glutamine, non-essential amino acids, vitamins, growth factors, low-molecular-weight compounds, antibiotics, antioxidants, pyruvic acid, buffers, inorganic salts, cytokines, and the like.

A cytokine selected from the group consisting of BMP4 (bone morphogenetic protein 4), VEGF (vascular endothelial growth factor), bFGF (basic fibroblast growth factor), SCF (stem cell factor), TPO (thrombopoietin), and FLT3L (Flt3 ligand) may be further added to a culture medium used for producing hematopoietic stem cells. The concentrations thereof are, for example, 1 to 100 ng/mL for BMP4, 1 to 100 ng/mL for VEGF, 1 to 100 ng/mL for bFGF, and 10 to 100 ng/mL for SCF, 1 to 100 ng/mL for TPO, and 1 to 100 ng/mL for FLT3L.

A TGF β inhibitor may be added to a culture medium of hematopoietic stem cells. A "TGF β inhibitor" is a low-molecular inhibitor interfering with signaling of TGF β family. Examples thereof include SB431542 and SB202190 (R. K. Lindemann et al., Mol. Cancer 2: 20 (2003)), SB505124 (GlaxoSmithKline), NPC30345, SD093, SD908, and SD208 (Scios), as well as LY2109761, LY364947, and LY580276 (Lilly Research Laboratories). The concentration thereof in a culture medium is preferably 0.5 to 100 µM.

iPS cells may be co-cultured with feeder cells such as C3H10T1/2 (Takayama N., et al. J Exp Med. 2817-2830, 2010), or heterologous stromal cells (Niwa A et al. J Ce11 Physiol. 2009 Nov; 221 (2): 367-77), and the like.

The method for culturing iPS cells in the production of hematopoietic stem cells may be adhesion culture or suspension culture. However, suspension culture is preferable. For example, iPS cells can be subjected to suspension culture after releasing colonies cultured to 80% confluency in a dish used and dissociating them into individual cells. Examples of the methods for isolating iPS cells include physical methods using a cell scraper or the like, and methods using a dissociation solution with protease activity and collagenase activity (e.g., Accutase^{®} and Accumax^{®}, etc.), or a dissociation solution with collagenase activity.

Suspension culture is a way of culturing cells wherein the cells do not adhere to a culture vessel. Suspension culture can be performed by using a culture vessel which is not artificially treated (e.g., coating with an extracellular matrix, etc.) for improving adhesion with cells, or a culture vessel which is artificially treated to suppress adhesion (e.g., coating with polyhydroxyethyl methacrylate (poly-HEMA) or nonionic surfactant polyol (Pluronic F-127, etc.). However, it is not particularly limited. During a suspension culture, it is preferable to form and culture embryoid bodies (EB). When suspension-culturing embryoid bodies to obtain hematopoietic stem cells, it is preferable to dissociate them into individual cells and then perform the adhesion culture.

Hematopoietic stem cells can also be prepared from cystoid-like structures (also referred to as iPS-sac) obtained by culturing iPS cells. Here, the "cystoid-like structure" is an iPS cell-derived three-dimensional bursal structure (with internal space), formed of groups of endothelial cells and the like, and containing hematopoietic stem cells inside.

The temperature condition during culture for producing hematopoietic stem cells from iPS cells is not particularly limited, but for example about 37°C to about 42°C, and preferably about 37°C to about 39°C. In addition, the culture period can be appropriately determined by a person skilled in the art while monitoring the number of hematopoietic stem cells and the like. The number of culturing days is not particularly limited as long as hematopoietic stem cells can be obtained, but is, for example, at least 6 days or more, 7 days or more, 8 days or more, 9 days or more, 10 days or more, 11 days or more, 12 days or more, 13 days or more, or 14 days or more, and preferably 14 days. Along culture period does not cause any problem in the production of hematopoietic stem cells. Besides, a low-oxygen condition is also possible. In one aspect of the present invention, examples of oxygen concentration as low-oxygen conditions include 15%, 10%, 9%, 8%, 7%, 6%, 5%, or less.

"CD4/CD8 double-positive T cells" are the cells among T cells wherein the surface antigens CD4 and CD8 are both positive (CD8⁺ CD4⁺). Since the T cells can be recognized by the fact that surface antigens CD3 and CD45 are positive, CD4/CD8 double-positive T cells can be identified as the cells wherein CD4, CD8, CD3, and CD45 are positive. CD4/CD8 double-positive T cells can be differentiated into CD4 single-positive cells or CD8 single-positive cells by induction.

CD4/CD8 double-positive T cells can be produced by a method comprising a step for culturing hematopoietic stem cells in a culture medium added with p38 inhibitor and/or SDF-1.

A "p38 inhibitor" is defined as a substance that inhibits the function of p38 protein (p38MAP kinase). Examples of p38 inhibitors include a chemical inhibitor of p38, a dominant negative mutant of p38, or a nucleic acid encoding the same, however, they are not limited thereto.

Examples of chemical inhibitors of p38 include SB 203580 (4-(4-fluorophenyl)-2-(4-methylsulfonylphenyl)-5-(4-pyridyl)-1H-imidazole) and derivatives thereof, SB202190 (4-(4-fluorophenyl)-2-(4-hydroxyphenyl)-5-(4-pyridyl)-1H-imidazole) and derivatives thereof, SB239063 (trans-4-[4-(4-fluorophenyl)-5-(2-methoxy-4-pyrimidinyl)-1H-imidazole-1-yl] cyclohexanol) and derivatives thereof, SB220025 and derivatives thereof, PD169316, RPR200765A, AMG-548, BIRB-796, SCIO-469, SCIO-323, VX-702, or FR167653, however, they are not limited thereto. These compounds are commercially available. For example, SB203580, SB202190, SC239063, SB220025, and PD169316 are available from Calbiochem, and SCIO-469 and SCIO-323 are available from Scios etc. A p38 inhibitor is added to a culture medium in a range of, for example, about 1 µM to about 50 µM.

Examples of dominant negative mutants of p38 include p38T180A in which threonine at position 180 located in the DNA binding region of p38 is point-mutated to alanine, and p38Y182F in which tyrosine at position 182 of p38 in human and mouse is point-mutated to phenylalanine, and the like.

SDF-1 (stromal cell-derived factor 1) may be not only SDF-1α or its mature form, but also isoforms such as SDF-1β, SDF-1γ, SDF-16, SDF-1ε, or SDF-1ϕ, or mature forms thereof, or a mixture thereof at any proportion. Preferably, SDF-1α is used. SDF-1 may also be referred to as CXCL-12 or PBSF.

SDF-1 may be substituted, deleted and/or added with one or several amino acids in its amino acid sequence as long as having the activity as the chemokine. Similarly, sugar chains may be substituted, deleted and/or added. An amino acid mutation is acceptable if at least four cysteine residues (Cys30, Cys32, Cys55, and Cys71 in case of human SDF-1α) are retained in SDF-1, and it has the identity of 90% or more to the amino acid sequence of natural form. SDF-1 may be that of a mammal such as a human, a nonhuman mammal such as monkey, sheep, bovine, horse, pig, dog, cat, rabbit, rat, or mouse, etc. For example, the protein registered in GenBank with registration number of NP_954637 can be used as human SDF-1α, and the protein registered in GenBank with registration number of NP_000600 can be used as SDF-1β.

As SDF-1, those which are commercially available may be used. Those which are purified from natural products may be used. Alternatively, those which are produced by peptide synthesis or genetic engineering techniques may also be used. SDF-1 is added to a culture medium in a range of, for example, about 10 ng/mL to about 100 ng/mL.

A culture medium used for producing CD4/CD8 double-positive T cells is not particularly limited. It can be prepared by using a culture medium for culturing animal cells as a basal culture medium, and adding p38 inhibitor and/or SDF-1, and furtherly vitamin C. The types of vitamin C used in the production of CD4/CD8 double-positive T cells are, for example, as described above, and the concentration thereof is, for example, 5 to 200 µg/mL. Examples of basal culture media include Iscove's Modified Dulbecco's Medium (IMDM), Medium 199, Eagle's Minimum Essential Medium (EMEM), αMEM medium, Dulbecco's modified Eagle's Medium (DMEM), Ham's F12 medium, RPMI 1640 medium, Fischer's Neurobasal Medium (Life Technologies), and a mixed medium thereof. A culture medium may be added with serum or may be serum-free. If necessary, a basal medium may contain one or more substances selected from, for example, albumin, insulin, transferrin, selenium, fatty acids, trace elements, 2-mercaptoethanol, thioglycerol, lipids, amino acids, L-glutamine, non-essential amino acids, vitamins, growth factors, low-molecular-weight compounds, antibiotics, antioxidants, pyruvic acid, buffers, inorganic salts, cytokines, and the like.

A cytokine selected from the group consisting of SCF, TPO (thrombopoietin), FLT3 L, and IL-7 may be further added to a culture medium used for producing CD4/CD8 double-positive T cells. The concentrations thereof are, for example, 10 to 100 ng/mL for SCF, 10 to 200 ng/mL for TPO, 1 to 100 ng/mL for FLT3L, and 1 to 100 ng/mL for IL-7.

Hematopoietic stem cells may be cultured by adhesion culture or suspension culture. However, adhesion culture is preferable. In the case of adhesion culture, a culture vessel may be coated and used. Examples of coating agents include Matrigel (Niwa A, et al. PLos One. 6 (7): e22261, 2011), collagen, gelatin, laminin, heparan sulfate proteoglycan, retronectin, Fc-DLL4 or entactin, and combinations thereof.

The condition of culture temperature for culturing hematopoietic stem cells to produce CD4/CD8 double-positive T cells is not particularly limited, but preferably about 37°C to about 42°C, and more preferably about 37°C to about 39°C. In addition, the culture period can be appropriately determined by a person skilled in the art while monitoring the number of CD4/CD8 double-positive T cells or the like. The number of culturing days is not particularly limited as long as CD4/CD8 double-positive T cells can be obtained, but is, for example, at least 10 days or more, 12 days or more, 14 days or more, 16 days or more, 18 days or more, 20 days or more, 22 days or more, or 23 days or more, and preferably 23 days.

The resulting CD4/CD8 double-positive T cells may be isolated and used, or used as a cell population containing other cell species. In the case of isolation, methods well known to those skilled in the art can be used. For example, a method of labeling with antibodies against CD4, CD8, CD3 and/or CD45, and isolating using a flow cytometer, or a purification method using an affinity column in which a desired antigen is immobilized can be exemplified.

"CD8 single-positive T cells", that is, mature T cells are the cells among T cells wherein the surface antigen CD8 is positive (CD8⁺ CD4⁻), and are also referred to as cytotoxic T cells. Since the T cells can be recognized by the fact that surface antigens CD3 and CD45 are positive, CD8 single-positive T cells can be identified as the cells wherein CD8, CD3 and CD45 are positive while CD4 is negative.

CD8 single-positive T cells can be produced by a method comprising a step for culturing CD4/CD8 double-positive T cells in a culture medium added with a corticosteroid.

The corticosteroid is preferably a glucocorticoid or a derivative thereof, and examples thereof include cortisone acetate, hydrocortisone, fludrocortisone acetate, prednisolone, triamcinolone, methylprednisolone, dexamethasone, betamethasone, or beclomethasone dipropionate. Preferably, the corticosteroid is dexamethasone. The concentration thereof in a culture medium is, for example, 1 to 100 nM.

A culture medium used for producing CD8 single-positive T cells is not particularly limited. It can be prepared by using the culture medium for culturing animal cells as a basal culture medium, and adding a corticosteroid thereto. Examples of basal culture media include Iscove's Modified Dulbecco's Medium (IMDM), Medium 199, Eagle's Minimum Essential Medium (EMEM), αMEM medium, Dulbecco's modified Eagle's Medium (DMEM), Ham's F12 medium, RPMI 1640 medium, Fischer's Neurobasal Medium (Life Technologies), and a mixed culture medium thereof. A culture medium may be added with serum or may be serum-free. If necessary, a basal medium may contain one or more substances selected from, for example, albumin, insulin, transferrin, selenium, fatty acids, trace elements, 2-mercaptoethanol, thioglycerol, monothioglycerol, lipids, amino acids, L-glutamine, non-essential amino acids, vitamins, growth factors, low-molecular-weight compounds, antibiotics, antioxidants, pyruvic acid, buffers, inorganic salts, cytokines, and the like.

The culture medium used in the production of CD8 single-positive T cells preferably further contains anti-CD3 antibody, vitamin C, or cytokine. Examples of the cytokine include IL-2, IL-7, IL-15, IL-21, and the like.

The anti-CD3 antibody is not particularly limited as long as it is an antibody that specifically recognizes CD3. Examples thereof include antibodies produced from OKT 3 clones. The concentration of the anti-CD3 antibody in a culture medium is, for example, 10 to 1000 ng/mL.

The vitamin C used in the production of CD8 single-positive T cells is, for example, those described above, and can be used in the same conditions as described above.

The concentrations of cytokines in a culture medium used in the production of CD8 single-positive T cells are, for example, 10 to 1000 U/mL for IL-2, and 1 to 100 ng/mL for IL-7.

The temperature condition during the culture of CD4/CD8 double-positive T cells for producing CD8 single-positive T cells is not particularly limited, but for example, preferably about 37°C to about 42°C, and more preferably about 37°C to about 38°C. In addition, the culture period can be appropriately determined by a person skilled in the art while monitoring the number of CD8 single-positive T cells and the like. The number of culturing days is not particularly limited as long as CD8 single-positive T cells can be obtained, but is, for example, at least 1 day or more, 2 days or more, 3 days or more, 4 days or more, or 5 days or more, and preferably 3 days.

### [Preparation of cDNAs encoding TCR]

In the present invention, the preparation of cDNAs encoding a TCR α chain and a β chain respectively is preferably performed from individual cells. T cells collected from subjects are T cell populations generally with genetic diversity. However, an antigen or peptide sequence recognized by TCR of individual cells is determined, and antigen specificity of individual cells is different. In order to select the TCR that is optimal for each tumor-related antigen, that is, the TCR that has high reactivity to each tumor-related antigens, it is preferable to prepare cDNAs from individual cells.

From T cells obtained from subjects, T cell populations responsive to tumor-related antigens may be isolated as a single cell, such as by a cell sorter, using CD106 as a marker. In addition to CD106, cell surface CD137 may be used as a marker to isolate cells. Examples of known methods for isolating human T cells include flow cytometry using an antibody against T cell surface markers such as CD106, CD3, and CD137 and a cell sorter. Gene cloning can be performed from the obtained single T cells using PCR method, and cDNAs encoding a TCR α chain and a β chain respectively can be amplified.

### [Construction of vectors]

The PCR fragment amplified using the cDNAs of isolated TCR as a template can be incorporated into a viral vector or a non-viral vector using, for example, Gibson Assembly System. Specifically, a gene in which the isolated TCR α chain gene and TCR β chain gene are linked via the T2A sequence is linked downstream of the ubiquitin promoter. Further downstream thereof, EGFR (EGFRt, truncated EGFR) excluding the ligandbinding site and intracellular domain for the IRES (internal ribosome entry site) sequence or a marker gene such as CD19 lacking an intracellular domain are linked. The construct is incorporated into a viral vector or a non-viral vector. As the vector, a viral vector and a non-viral vector can be used. However, a non-viral vector is preferable. As the non-viral vector, a transposon vector is preferable, and a piggyBac^{®} transposon vector is more preferable. Compared to conventional viral vector method, the transposon method is an inexpensive and safe transfection method of next generation.

### [Introduction of TCR cDNAs into cells]

As a method of introducing a cDNA pair encoding a TCR α chain and a β chain respectively to iPS cell clones derived from non-T non-B cells or monocytes or T cells; hematopoietic stem cells differentiated from the iPS cell clones; immature T cells differentiated from the hematopoietic stem cells, or mature T cells differentiated from the immature T cells, either a method using a viral vector or a non-viral vector, or a replacement of TCR using genome editing technique can be adopted. Examples of the viral vectors include viral vectors such as lentivirus, retrovirus, adenovirus, adeno-associated virus, herpes virus and Sendai virus, etc. and animal cell-expressing plasmid. However, retrovirus or lentivirus are preferable. When a retroviral or a lentiviral infection is carried out, a spin infection method or the like is preferably used. When a non-viral vector is used, a transposon method is preferable. For the replacement of TCR using genome editing technique, CRISPR/Cas9 method, CRISPR/MAD method, and CRISPR/CAS3 method may be used. Examples of transfection methods of non-viral vector or transfection methods of guide RNA and donor DNA for genome editing include lipofection method, liposome method, calcium phosphate coprecipitation method, DEAE dextran method, microinjection method, and electroporation method. PCR products can be directly introduced into cells without using a vector. An electroporation method is preferably used for introducing transposon vectors or PCR products into cells. As a electroporation apparatus, the transfection device of ExPERT^{®}system (MaxCyte) is preferable. As methods of introducing cDNA pairs respectively encoding TCR α chain and β chains, genome editing techniques such as CRISPR/Cas9 and TALEN may be used. The introduction of the TCR gene by CRISPR/Cas9 is accomplished, for example, by introducing a guide RNA designed in the genes of the endogenous TCR α chain and β chain (guide RNA for the sense strand and guide RNA for the antisense strand), as well as genes linking the target TCR α chain and β chain gene with the homologous recombination portion of the endogenous TCR α chain to the 5' side and the 3' side into target cells together with the vector encoding Cas9. In this case, endogenous promoters and enhancers are used for the expression of the TCR α chain and β chain gene of interest.

Examples of the promoters used in the expression vector for introducing the cDNA pair into the cells include EF1α promoter, SRα promoter, SV40 promoter, LTR promoter, CMV promoter, RSV promoter, HSV-TK promoter, ubiquitin promoter, and the like. These promoters may be capable of controlling the expression of genes inserted downstream of the promoter depending on the presence or absence of a drug such as tetracycline. An expression vector can comprise enhancer, poly A addition signal, selection marker gene (e.g., neomycin resistance gene), SV40 replication origin, and the like, in addition to the promoter.

By culturing the iPS cell clones into which the cDNA pair is introduced, hematopoietic stem cells differentiated from the iPS cell clones, immature T cells differentiated from the hematopoietic stem cells, or mature T cells differentiated from the immature T cells, under the culture conditions such as the above-described culture medium, culture medium composition, culture temperature, etc., the regenerated T cells expressing the TCR consisting of the α chain and the β chain that respectively encoding the cDNA pair can be obtained. In this culture, the proliferation of the cells is preferably carried out in the presence of feeder cells and PHA (phytohemagglutinin), in the presence of a RetroNectin^{®} and anti-CD3 antibody, or in the presence of anti-CD3 antibody and anti-CD28 antibody. The feeder cells are preferably autologous or allogeneic peripheral blood mononuclear cells. The "autologous" means that the subjects from whom peripheral blood mononuclear cells and the iPS cell clones derive are the same. The "allogeneic" means that the subjects from whom peripheral blood mononuclear cells and the iPS cell clones derive are different.

The iPS cell, the hematopoietic stem cell, the immature T cell, or the mature T cell into which the cDNA pair has been introduced may be cloned. For clone screening, it is preferable to perform cloning with a colony pick-up method. The colony pick-up methods include, but are not limited to, methods of using a pipetman under a microscope, limiting dilution methods, and methods using a fully automatic colony picker.

Whether or not the target TCR is expressed can be confirmed, for example, based on the repertoire analysis of TCR β gene, using a TCRV β analysis kit (Beckman Coulter, catalog number IM-3497). Since a marker gene such as EGFRt (truncated EGFR) and CD19t (truncated CD19) is incorporated into the vector, expression of the TCR α chain and the β chain can be speculated by analyzing the expression of the marker gene.

The iPS cell clones, the hematopoietic stem cells, the immature T cells, or the mature T cells may construct a master cell bank after expanded culture. Here, the "master cell bank" referred to here is a collection of the iPS cell clones, the hematopoietic stem cells, the immature T cells, or the mature T cells, which are dispensed into independent cell storage containers for each single pool of single clones. In general, it is preferable to proliferate the cells within a range in which the properties of the cells do not change, and dispense the cells into a plurality of cell preservation containers. The cells preserved in a master cell bank serve as starting materials for producing regenerated T cells by transfecting TCR genes or CAR (chimeric antigen receptor) genes. For each production of the regenerated T cells, a cell storage container containing the required number of the cells can be taken from the master cell bank. Therefore, the regenerated T cells of the present invention can be repeatedly supplied with the same quality.

In the master cell bank, it is preferable to cryopreserve the cells dispensed into a cell storage container. Methods for cell cryopreservation are well known to those skilled in the art. For example, a single clone of cells after expanded culture can be harvested and washed with a buffer solution or a culture medium, enriched by centrifugation or the like after the cell number is counted, suspended in a medium for cryopreservation (e.g., a culture medium containing 10% DMSO), and then cryopreserved at a low temperature. For example, a master cell bank can be constructed by accumulating 200 to 1000 cell storage containers each containing 1×10⁶ to 1×10⁷ cells. The master cell bank of the present invention can be preserved in any facility or storage capable of cryopreservation. In case of cryopreservation, the storage temperature is not particularly limited as long as it is suitable for the preservation of cells. Examples thereof include -20°C, -80°C, and - 120°C to -196°C, preferably -150°C or lower.

### [Production of regenerated T cells from iPS cells]

It is said that there are 5 to 10 TCR pairs corresponding to tumor-related antigens per epitope. Each TCR pair is predicted to have different sensitivity to point mutant of tumor-related antigens. Therefore, for example, even when a mutant of point mutation occurs in a tumor-related antigen, there is a high possibility that a TCR capable of recognizing the epitope is present, and regenerated T cells specific to the antigen can be quickly prepared for the mutant of point mutant. In addition, even when the tumor-related antigen has completely changed as a result of the mutation, even when a resistance occurs in cancer treatment with T cell replacement therapy, patient's reactivity to multiple tumor antigens can be analyzed, and production of TCRs that respond to these tumor-related antigens can be started from already obtained iPS cells that have a high ability to differentiate into T cells or differentiated cells derived from the iPS cells. Therefore, regenerated T cells can be produced quickly.

### [T cells as TCR acquisition source]

T cells which are acquisition source of TCR pairs corresponding to tumor-related antigens are preferably collected from tumor tissues. The harvested T cells may undergo cell isolation using CD106 as a marker after the tumor-responsive cell population is amplified by co-culture with tumor tissue *in vitro.*

When the subject from whom T cells for obtaining TCR pairs are collected, and the subject to be treated of cancer with a replacement therapy using regenerated T cells are separate individuals, it is not necessary to collect antigen-specific T cells from the targeted subject of treatment. Therefore, since the T cells for obtaining TCR pairs can be collected in advance at any time separately from the treatment time of a replacement therapy using regeneration T cells, the replacement therapy using regeneration T cells can be started quickly.

When the subject from whom T cells for obtaining TCR pairs are collected, and the subject to be treated of cancer with a replacement therapy using regenerated T cells are separate individuals, it is necessary to confirm that the obtained TCR pair does not show an alloreaction to the cells of the subject to be treated. The confirmation is performed by known methods. For example, it can be confirmed by mixed lymphocyte culture (MLR) or the like that T cells with the obtained TCR pairs do not respond to the cells of a subject to be treated. Alternatively, it is confirmed by that the T cells with the obtained TCR pairs are not responsive to an antigen in which one or more residues of the constituent amino acids of the antigen are substituted with other amino acids.

### [Pharmaceutical compositions]

The pharmaceutical compositions containing the regenerated T cells of the present invention can be used to treat a targeted subject of cancer treatment. The pharmaceutical compositions of the present invention can be produced by methods commonly used in the field of formulation technology, such as the methods described in the Japanese Pharmacopoeia. The pharmaceutical compositions of the present invention may contain pharmaceutically acceptable additives. Examples of the additives include a cell culture medium, physiological saline, a suitable buffer (e.g., phosphate buffer), and the like.

The pharmaceutical compositions of the present invention can be produced by suspending regenerated T cells in physiological saline or a suitable buffer (e.g., phosphate buffer). In order to exhibit a desired therapeutic effect, one dose preferably contains, for example, 1×10⁷ or more cells. More preferably, the content of the cells is 1×10⁸ or more, and further more preferably 1×10⁹ or more. The content of the cells can be appropriately adjusted in consideration of sex, age, body weight of the subject, state of affected part and the cells, and the like. For protecting the cells, the pharmaceutical compositions of the present invention may contain dimethyl sulfoxide (DMSO) and serum albumin etc., beside regenerated T cells. In addition, for preventing bacteria from being mixed, they may contain vitamins, cytokines, and the like in order to promote activation and differentiation of antibiotics etc. and cells. Furthermore, the pharmaceutical compositions of the present invention may contain other pharmaceutically acceptable ingredients (for example, a carrier, an excipient, a disintegrant, a buffer, an emulsifier, a suspension, an analgesic agent, a stabilizer, a preservative, an antiseptic agent, physiological saline, or the like).

The pharmaceutical compositions containing the regenerated T cells of the present invention as an active ingredient can be cryopreserved. In case of cryopreservation, the storage temperature is not particularly limited as long as it is suitable for the preservation of cells. Examples thereof include -20°C, -80°C, and -120 to -196°C, preferably -150°C or lower. In case of cryopreservation, the cells are preferably preserved in an appropriate container such as a vial and a bag for cryopreservation. Procedures for minimizing the risk of cell damage during freezing and thawing of regenerated T cells are well known to those skilled in the art.

In the cryopreservation of the regenerated T cells of the present invention, the T cells are harvested from a culture medium, washed with a buffer solution or a culture medium, enriched by centrifugation or the like after the cell number is counted, suspended in a medium for cryopreservation (e.g., a culture medium containing 10% DMSO), and then cryopreserved at a low temperature. Regenerated T cells may be preserved clone by clone or as a mixture of clones. The pharmaceutical compositions containing the regenerated T cells of the present invention may contain, for example, 5×10⁴ to 9×10¹⁰ regenerated T cells per container such as a vial and a bag for cryopreservation. However, it can be changed in accordance with type of the target cancer, subject, administration route, and the like.

Examples of the administration route of the pharmaceutical compositions containing the regenerated T cells of the present invention includes infusion, intratumoral injection, arterial injection, portal vein injection, intraperitoneal administration, and the like. However, the administration route is not limited thereto as long as the regenerated T cells, which are the active ingredients in the pharmaceutical compositions of the present invention, are delivered to an affected part. As a schedule of administration, both single administration and multiple administration are possible. Regarding the period of a multiple administration, for example, a method of repeating administration once every 2 to 4 weeks or a method of repeating administration once every six months to a year can be adopted. In the preparation of a schedule of administration, sex, age, body weight, conditions, and the like of a targeted patient can be considered.

In the case where the pharmaceutical compositions containing the regenerated T cells of the present invention are used for the prevention or treatment of cancer in a cancer patient, when the TCR on the regenerated T cells is allogeneic, a pharmaceutical composition containing the regenerated T cells introduced with the TCR that do not exhibit alloreaction to the normal cells of cancer patients is used in order to avoid *in vivo* alloreaction in cancer patients.

The pharmaceutical compositions of the present invention are used for prevention or treatment of cancer. Examples of the cancer include hepatoma, hepatoblastoma, gastric cancer, esophageal cancer, lung cancer, pancreatic cancer, renal cell cancer, breast cancer, ovarian cancer, skin cancer, such as malignant melanoma, bladder cancer, head and neck cancer, uterine cancer, cervical cancer, glioblastoma, prostate cancer, neuroblast, chronic lymphocytic leukemia, thyroid papilla cancer, colon cancer, brain tumor, sarcoma, or B-cell non-Hodgkin's lymphoma. The pharmaceutical compositions of the present invention can be suitably used for highly immunogenic cancers such as hepatoma, hepatoblastoma, colon cancer, lung cancer, and malignant melanoma. An "immunogenic cancer" is a cancer that is highly capable of inducing an immune response, and refers to the cancer wherein many genetic mutations (1000 or more or 5000 or more per cell) are found in their cells, the immune checkpoint inhibitors show efficacy, and many immune cells are infiltrating the tumor tissue (many immune cells are recognized by histopathological staining).

### EXAMPLES

The present invention will be described below according to examples. However, the present invention is not limited to the following examples.

### Example 1

### [Production of regenerated T cells from iPS cells in which non-T non-B cells or monocytes of peripheral blood have been reprogramed]

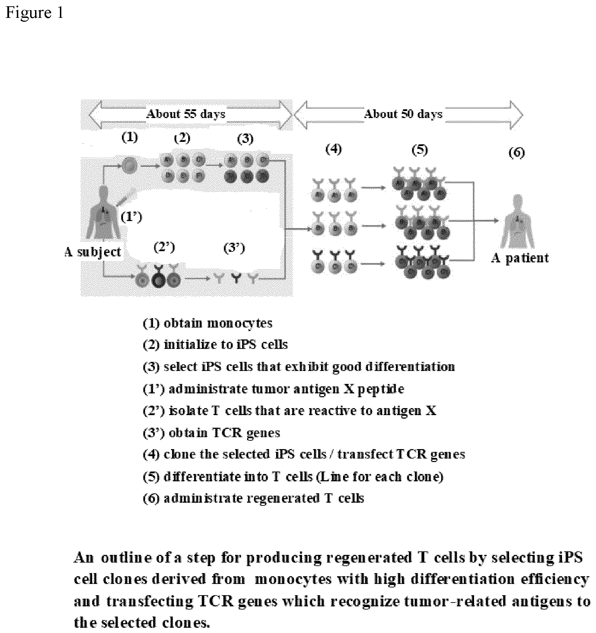

FIG. 1 shows a step for producing the regenerated T cells of the present invention. Mononuclear cells in the tumor tissue of a cancer patient (subject) undergoing treatment are collected, T cells that specifically react with the tumor antigen were isolated as CD106-positive T cells, and TCR genes were isolated from the CD106-positive T cells (FIG. 1-(2')). The TCR gene which is expected to have a higher therapeutic effect was selected according to the binding ability specific to the tumor antigen (FIG. 1-(3')). When tumor recurrence and mutation in tumor cells occur in a patient undergoing treatment, effective TCR gene for the treatment can be selected again. In order to achieve sufficient therapeutic effects, it is important to acquire many types of TCR (TCR repertoire).

Separately, iPS cells were produced from the non-T non-B cells or monocytes of peripheral blood of the patient (FIGS. 1 (1) and (2)). Next, iPS cell clones with good differentiation efficiency to T cells were selected from the obtained iPS cells (FIG. 1 (3)). It was confirmed that this selection significantly reduced the variation in induction efficiency to T cells due to differences in iPS cell clones, and improved the robustness and efficiency of the production of regenerated T cells. The above selected TCR gene group that specifically responded to tumor antigens was expressed in iPS cell clones with good differentiation efficiency into the T cells (FIG. 1 (4)), and differentiation into mature T cells and proliferation were performed for each of the selected TCR genes (FIG. 1 (5)). The mature T cells were cytotoxic T cells that were specific to tumor antigens, and were regenerated T cells. These regenerated T cells were administered to a cancer patient (FIG. 1 (6)).

In the method of the present invention, since the step for producing iPS cells (FIGS. 1-(1) to (3)) and the step for obtaining the TCR genes that react with tumor antigen (FIGS. 1-(2') and (3')) can be performed in parallel, these steps can be completed in a shorter period (about 55 days) as compared with a case where these steps are sequentially performed.

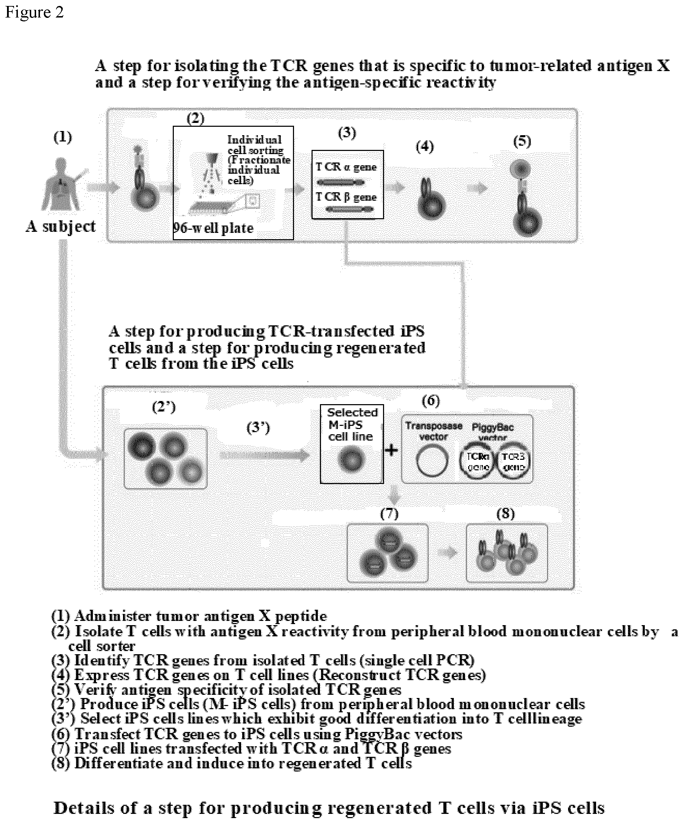

The above will be described in more detail. The isolation of TCR genes that are specific to tumor antigen and verification of antigen-specific reactivity were performed as below (see the upper diagram of FIG. 2). After co-culturing tumors (specimens) isolated from cancer patients (subjects) and CD8-positive cytotoxic T cells, single cell sorting of activated CD106-positive cytotoxic T cells (CD8-positive and CD106-positive cells) was performed with a cell sorter (FIG. 2-(2)). A TCR gene pair (TCR α chain gene and TCR β chain gene) was isolated from isolated individual cells by a single cell PCR, sequence analysis was performed on the isolated TCR gene pair, and the type of the T cells that were reactive to tumor antigen (TCR repertoire) and the appearance frequency thereof were analyzed (FIG. 2-(3)). The TCR gene pair isolated was expressed in a TCR gene deficient T cell line incorporating a reporter gene, and the TCR gene complex was reconstructed (FIG. 2-(4)). Subsequently, the reactivity between reconstructed TCR gene complex and tumor (antigen-specific reactivity) was verified (FIG. 2-(5)). That is, when the T cell line expressing the TCR gene pair and the targeted tumor cells were co-cultured, but the expressed TCR and the tumor were antigen-specifically bound, the TCR gene signal was activated, and the reporter gene incorporated in the downstream of the target gene promoter was expressed. After that, the antigen-specific reactivity was verified by determining the activity of reporter gene, and assessing the binding of the tumor antigen-specific TCR. Through the above steps, from among the TCR gene pairs that exhibited specific reactivity to tumor antigen, multiple ones with a high appearance frequency were selected, and used as transgenes to non-T non-B cell or monocyte-derived iPS cells (M-iPS cells), which will be described later.

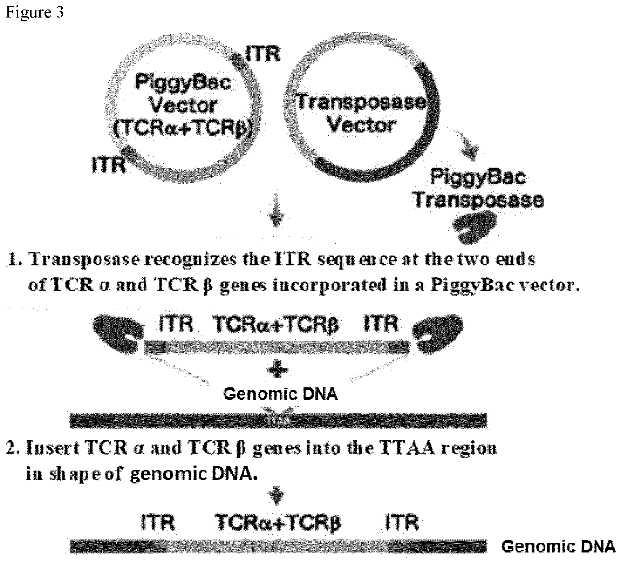

The production of TCR transfected iPS cells and regenerated T cells was carried out as below (see the lower diagram of FIG. 2). iPS cells were produced from peripheral blood mononuclear cells of a cancer patient (FIG. 2-(2 ')). The M-iPS cells described in the figure were the iPS cells that were not derived from T cells, and were the cells in which the gene reconstitution of the TCR gene was not performed. Next, M-iPS cell lines with good differentiation efficiency into T cells were selected from the produced M-iPS cell line groups (FIG. 2-(3')). TCR genes that were specific to tumor antigen (both TCR α chain gene and TCR β chain gene, see FIG. 2-(3)) were transfected into the selected M-iPS cell line by a transposon (see FIG. 2-(6) and FIG. 3), and M-iPS cell lines expressing the TCR genes (TCR-iPS cell lines) were selected by a cell sorter (MACSQuant Tyto^{®} cell sorter, Miltenyi Biotec), using the expression of CD19, a marker molecule, as an indicator. Next, a hematopoietic stem precursor cells were induced by using embryoid method, and differentiation induction to mature T cells via immature T cells was performed by a stepwise differentiation induction method imitating *in vivo* T cell generation.

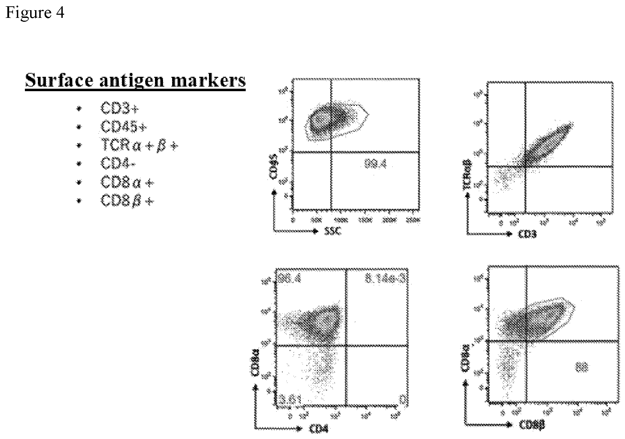

The phenotype of the regenerated T cells induced from iPS cells as described above (tumor antigen-specific CD8-positive cytotoxic T cells) was analyzed. The results of the analysis of the regenerated T cells according to the antigen marker on cell surface with a flow cytometer are shown in FIG. 4. The Regenerated T cells induced from iPS cells were confirmed to express CD45⁺ TCR αβ⁺ CD3⁺ CD4⁻ CD8αβ⁺, which was found to be expressed in mature cytotoxic T cells *in vivo.*

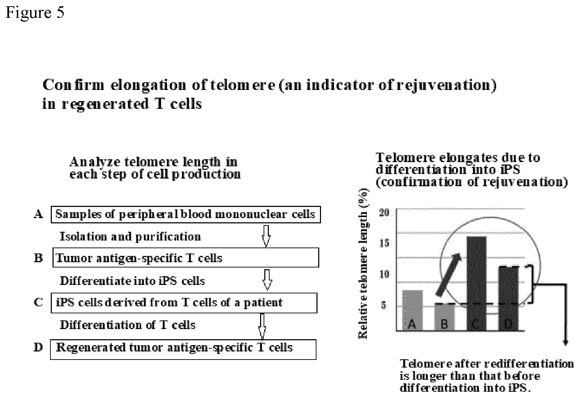

Results of analyzing telomere (an indicator of rejuvenation), a cell aging marker, regarding the regenerated T cells via the iPS cells of the present invention are shown in FIG. 5. Compared with the telomere length of the peripheral blood mononuclear cells of a patient (A) and the tumor antigen-specific T cells before regeneration (B), the telomere length of the iPS cells produced from the tumor antigen-specific T cells (C) was about three times, and that of the regenerated T cells of the present invention which were specific to tumor antigen (D) was about 2 times, and recovery in telomere length was confirmed in (C) and (D). That is, the improvement of cell aging is indicated in the regenerated T cells of the present invention.

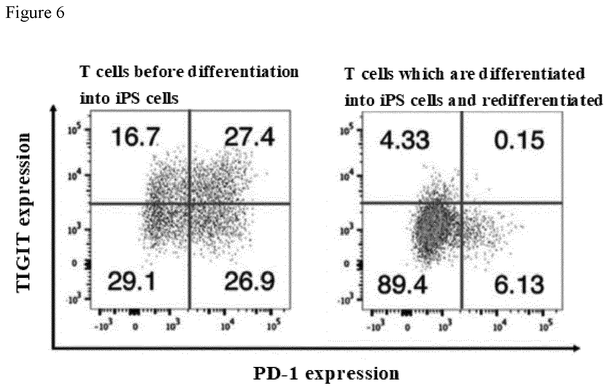

The regenerated T cells via iPS cells of the present invention were stained with anti-TIGIT antibody and anti-PD-1 antibody for the expression of PD-1, which was one of the cell exhaustion markers associated with immune checkpoints, and TIGIT molecule, and analyzed with a flow cytometer. For comparison, the tumor antigen-specific T cells before regeneration were also analyzed. The results are shown in FIG. 6. It was confirmed that the regenerated T cells of the present invention greatly reduced the expressions of PD-1 and TIGIT molecule compared to the tumor antigen-specific T cells before regeneration. Therefore, it is indicated that the regenerated T cells of the present invention have high cytotoxic activity.

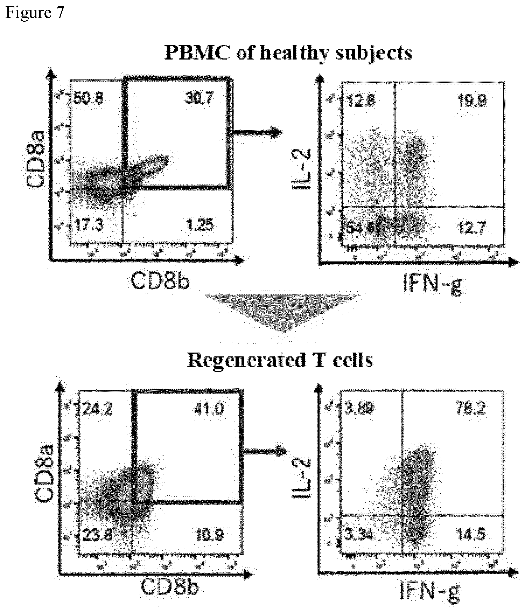

Cytokine-producing ability, which is an important indicator of cytotoxicity, was analyzed. Cytotoxic T cells in peripheral blood mononuclear cells (CD8 α chain/β chain double-positive cells) collected from healthy subjects and the regenerated T cells of the present invention were stimulated with PMA (phorbol 12-myristate 13-acetate) and ionomycin, and the amount of IL-2 and IFN-γ produced was compared. The results are shown in FIG. 7. It was conformed that the cells that produce IL-2 and IFN-γ were significantly increased in the regenerated T cells of the present invention, compared with those in the cytotoxic T cells obtained from healthy subjects.

### Example 2

### [Preparation of iPS cell clones]

Mononuclear cells were isolated from peripheral blood collected from patients with hepatoma or hepatoblastoma using mononuclear cell separation solution Lymphoprep^{®}. From the obtained mononuclear cells, CD19/CD20 positive B cells and CD3/CD4/CD8 positive T cells were removed using FACS or MACS beads to obtain non-T non-B cells or monocytes. The obtained non-T non-B cells or monocytes population were infected with Sendai virus (CytoTune^{®} 2.0) loaded with Yamanaka's four factors (Oct3/4, Sox2, Klf4, and c-Myc) and Sendai virus that had coded for SV40Tag, at an MOI (multiplicity of infection) of 5 to 20. It should be noted that the SV40 may be removed.

The obtained iPS cells consist of a number of iPS cell clones. For this reason, colonies were picked up and cloned. All cloned iPS cells were cryopreserved. Cloned iPS cells were cultured in a differentiation medium for about 10 days to induce hematopoietic stem cells, and CD34/CD43 double-positive hematopoietic stem cells were isolated. The isolated hematopoietic stem cells were cultured for about 21 days on a plate coated with FcDLL4, a fusion protein of DLL4 protein and Fc region of immunoglobulin, and differentiation induction into T cells was performed.

The frequency of obtaining immature cytotoxic T cells after the culture for 21 days was verified by the CD8 α chain/β chain double-positive rate, and the clones with the highest appearance frequency of the CD8 α chain/β chain double-positive cells was selected.

A master cell bank was constructed by expanded culture of several iPS cell clones with good differentiation efficiency into T cells in an iPS cell maintenance medium for two weeks, then dispensing them into a cell storage container, followed by cryopreservation.

### Example 3

### [Transfection of TCR genes to iPS cell clones]

To the iPS cell clones which was obtained in Example 2 and derived from non-T non-B cells or monocytes with good differentiation efficiency into T cells, a piggyBac vector having the genes encoding a TCR α chain and a β chain in which tumor antigen specificity had been confirmed was introduced using electroporation method. Then, the iPS cells expressing the target TCR α chain and β chain were isolated by a cell sorter using the expression of CD19, a marker molecule, as an indicator. The isolated iPS cells were cultured for about 10 days in a differentiation medium and CD34/CD43 double-positive hematopoietic stem cells were induced and isolated by a cell sorter. The isolated blood stem cells were cultured for about 21 days on a plate coated with FcDLL4, and differentiation induction into T cells was performed.

The CD8 α-chain / β-chain double-positive immature T cells obtained after the culture for 21 days were isolated and purified using a cell sorter. Then, the immature T cells were co-cultured in the presence of PHA (phytohemagglutinin) and peripheral blood mononuclear cells as feeder cells, in the presence of RetroNectin^{®} and anti-CD3 antibodies, or in the presence of anti-CD3 antibodies and anti-CD28 antibodies to induce mature cytotoxic T cells. These stimuli were carried out once or more. The performance of the obtained T cells was confirmed by the cytotoxic activity of the GPC3-specific target cells, IFN-γ production, and antigen binding ability.

### Example 4

### [Specificity of CD106 expression in tumor-cytotoxic T cells]

Tumor tissues were obtained from surgical specimens prior to intiation of treatment with anti-PD-1 antibodies in melanoma patients with whom the effects of anti-PD-1 antibodies was significant. Collagenase and DNase were added to the tumor tissue obtained and enzymatically treated by stirring at 37°C for 30 minutes to separate the cells. CD3-positive cells were sorted using BD FACSAria III^{®} cell sorter (BD Biosciences) from a cell mixture of the cells derived from tumor tissue and tumor infiltrating immune cells to obtain cells expressing TCR.

Each of the obtained T cells was sequenced using the 5 Prime Kit of Chromum system (10 X Genomics) and the V (D) J Enrichment Kit to perform single cell gene expression analysis for both gene expression and TCR sequence of marker candidates. HiSeq 3000 (Illumina) was used as the sequencer.

UMAP plots were created from the resulting fastg file using Python's umap library. The display of each gene was mapped onto the resulting UMAP plots.

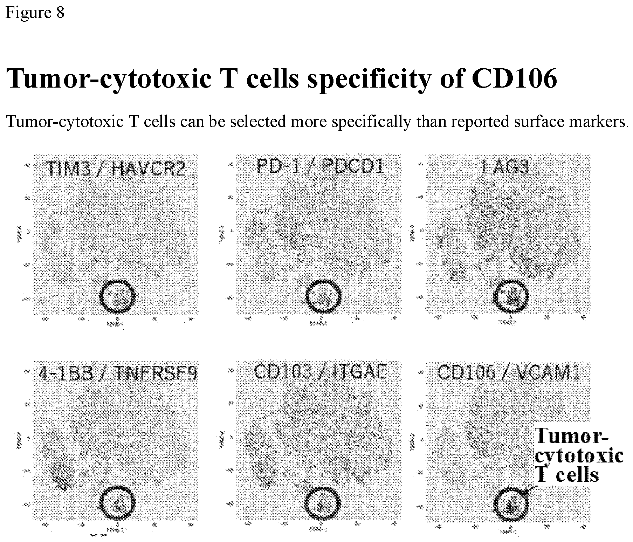

The results are shown in FIG. 8. In each panel of FIG. 8, cells in which the expression of the marker described in the panel is positive are shown colored. The cells are clustered by expression patterns, and the encircled portions correspond to tumor-cytotoxic T cells. It is understood that CD106 (VCAM1) positive cells are expressed in a population of tumor-cytotoxic T cells with high specificity. It is indicated that CD106 can be used as a selectable marker for tumor-cytotoxic T cells with greater specificity than surface markers other than CD106. TCR sequences were obtained in cells with more than 90% β chain and in cells with 60-90% α chain.

### Example 5

### [Tumor aggression of CD106-positive cell population]

Cells of tumor tissue were separated in the same way as that in Example 5. CD3 positive cells were sorted from the resulting cell mixture using BD FACSAria III. T cells expressing TCR were obtained as tumor infiltrating T cells. The tumor infiltrating T cells and cell lines established from autologous tumors as stimulants were co-cultured and IFN-γ production was compared to the cases without stimulation. IFN-γ production was measured by intracellular staining with anti-IFN-γ antibodies. Cell staining was performed using anti-PD-1 antibodies, anti-TIGIT antibodies, anti-LAG3 antibodies, and anti-CD106 antibodies. As negative control, the isotype control antibodies for anti-CD106 antibodies were used.

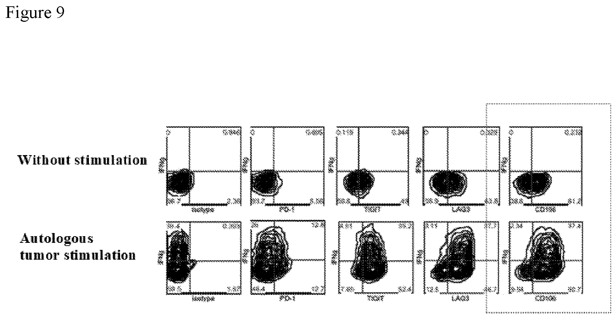

The results are shown in FIG. 9. It can be understood that IFN-γ is produced by stimulation with autologous tumor cell lines only in CD106-positive cell sub-populations, and IFN-γ is produced by autologous tumor stimulation in marker-positive cell sub-populations other than CD106, but since IFN-γ is also produced in the marker-negative cell sub-population, the specificity is inferior to CD106.

### Example 6

### [Transfection of TCR genes to mature T cells differentiated from iPS cell clones]

To mature T cells differentiated from the iPS cell clones which were obtained in Example 2 and derived from non-T non-B cells or monocytes with good differentiation efficiency into T cells via hematopoietic stem cells and immature T cells, genes (cDNAs) encoding a GPC3 antigen-specific TCR α chain and β chain were transfected using a piggyBac^{®} system in the same way as that in Example 3.

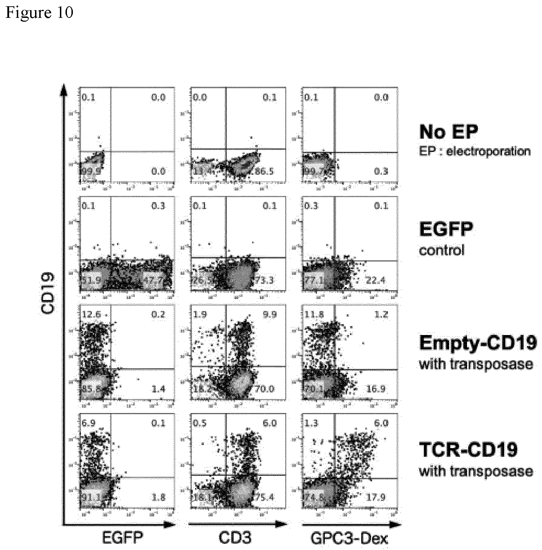

The results of analyzing the phenotype of the mature T cells derived from the transfected iPS cells with a flow cytometer are shown in FIG. 10. In FIG. 10, "No EP" shows the analytical results of the mature T cells used in transfection, and in which the WT1 antigen-specific TCR α chain and β chain are expressed. "EGFP" shows the analytical results of the mature T cells introduced with an expression vector incorporating a tracer gene (EGFP (enhanced green fluorescent protein) gene) as an indicator of transfection manipulation. "Empty-CD19" shows the analytical results of the mature T cells introduced with a PiggyBac^{®} transposon vector incorporating only an intracellularly defective human CD19 gene as a tracer gene and a transposon vector. "TCR-CD19" shows the analytical results of the mature T cells introduced with a PiggyBac^{®} transposon vector incorporating a GPC3 antigen-specific TCR α-chain β-chain gene and an intracellularly defective human CD19 gene in tandem.

In the iPS cell-derived mature T cells represented by "No EP", only CD3, a T cell marker, was detected. That is, the cells used for transfection were confirmed to be T cells.

In the iPS cell-derived mature T cells represented by "EGFP", expression of CD3 genes and EGFP genes was detected. That is, it can be seen that the transfection manipulation had been appropriately performed.

In the iPS cell-derived mature T cells represented by "Empty-CD19", the expression of CD3 gene and intracellular defective human CD19 gene, which is a tracer gene incorporated into piggyBac^{®} transposon vector was detected. That is, it can be seen that the transfection manipulation had been appropriately performed.

In the iPS cell-derived mature T cells represented by "TCR-CD19", in addition to expression of CD3 gene and intracellular defective human CD19 gene, which is a tracer gene incorporated into piggyBac^{®} transposon vector, the binding to GPC3 peptide/HLA complex (GPC3-Dex) recognized by GPC3 antigen-specific TCR α chain and β chain was detected. That is, it has been shown that by transfecting a GPC3 antigen-specific TCR α chain and β chain gene into iPS cell-derived mature T cells using a piggyBac^{®} system, a GPC3 antigen-specific TCR α chain and β chain expressed on the cells function as molecules that recognize a GPC3 peptide/HLA complex (GPC3-Dex). Therefore, it has been shown that by using novel tumor-related antigens (neoantigens) and TCR α chain and β chain genes which are specific to other tumor-related antigens as TCR α chain and β chain genes to be transfected into iPS cell-derived mature T cells, iPS cell-derived mature T cells that recognize these antigens can be produced.

### Example 7

### [Production of regenerated T cells from iPS cells in which peripheral blood T cells have been reprogramed]

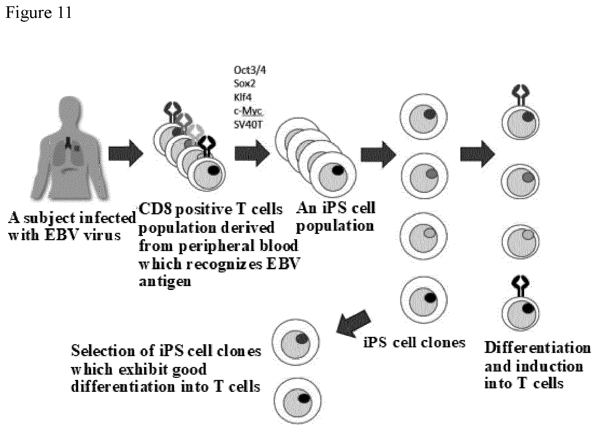

FIG. 11 shows a method for selecting the iPS cell clones that demonstrate high differentiation efficiency into T cells in the step for producing regenerated T cells from iPS cells in which peripheral blood T cells have been reprogramed. From the peripheral blood of a subject infected with EBV (Epstein-Barr virus), mononuclear cells were isolated. The isolated mononuclear cells were stimulated with EBV antigen in a test tube. The CD8-positive T cell populations that recognize the EBV antigen were isolated. To the isolated CD8-positive T cell populations, Yamanaka's four factors (Oct3/4, Sox2, Klf4, and c-Myc) and SV40T antigen were introduced using a Sendai virus vector to obtain iPS cell populations. From the obtained iPS cell populations, iPS cell clones were fractionated. For each clone, the differentiation potential to T cells was examined, and the iPS cell clones that demonstrate high differentiation efficiency into T cells were selected. T cells that recognize EBV antigens are the cells that are less likely to cause alloreaction even in a case of allografting.

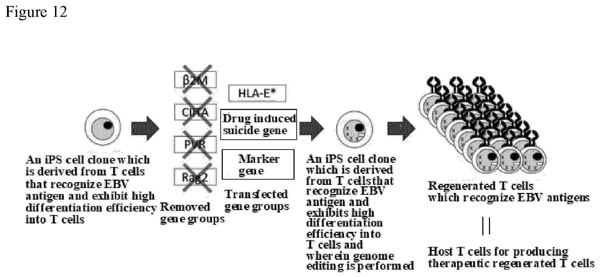

FIG. 12 shows a method for performing genome editing on iPS cell clones selected as the cells with high differentiation efficiency into T cells and producing regenerated T cells in the step for producing regenerated T cells from iPS cells in which peripheral blood T cells have been reprogramed. Regarding the iPS cell clones which are derived from CD8-positive T cells that recognize EBV antigen and demonstrate high differentiation efficiency into T cells, the β2M and CIITA genes involved in the expression of MHC class I and MHC class II, PVR genes involved in activation of natural killer (NK) cells, and Rag2 gene involved in rearrangement of T-cell receptor were deleted using CRISPR/Cas9. On the other hand, a fusion gene (HLA-E*) of β2M/binding peptide/HLA-E, which is an inhibitory ligand for NK cells, was expressed to make iPS cells immune to attack from T cells and NK cells of host. In this genome editing, cells can be removed after administration into a living body by expressing a suicide gene such as a drug-induced caspase 9 and/or a specific marker gene (EGFR (epidermal growth factor receptor), CD19, and CD20). After the genome editing, re-cloning was carried out. When the high differentiation efficiency to T cells was confirmed, the iPS cell-derived regenerated T cells may be preserved as clones to construct a master cell bank. The regenerated T cells differentiated and proliferated from iPS cells are host T cells for producing regenerated T cells for cancer treatment, and a master cell bank may be constructed by the host T cells. The host T cells can be used as a material for producing regenerated T cells into which TCR gene or CAR (chimeric antigen receptor) gene has been transfected. Since the host T cells are the T cells that recognize EBV antigen, they are unlikely to cause an alloreaction even when being transferred into a living body. "Host T cells" mean the T cells used as a starting material for producing a preventive or therapeutic agent for cancer containing regenerated T cells as an active ingredient, although the cells themselves are not subjected to treatment in patients.

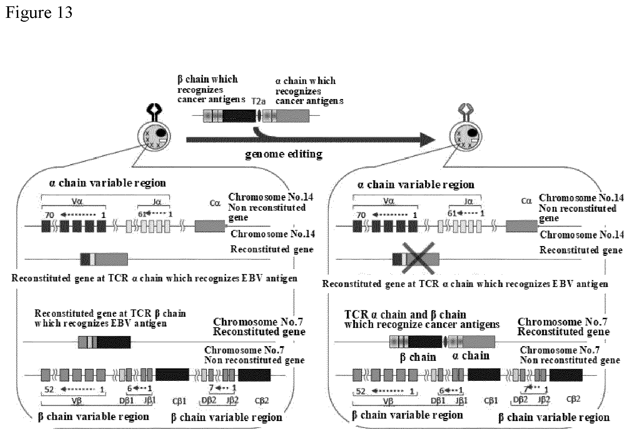

FIG. 13 shows a method for producing regenerated T cells which recognize cancer antigens from the host T cells. In the host T cells which recognize EBV antigens, the TCR β chain which recognize rearranged EBV antigen was replaced with a T2a-mediated conjugation of the TCR β chain and the TCR α chain, which recognize cancer antigens, respectively, by gene replacement according to genome editing using CRISPR/Cas9. On the other hand, the TCR α chain which recognizes EBV antigen was removed by genome editing using CRISPR/Cas9. According to the method described in FIG. 13, it was possible to produce regenerated T cells that recognize cancer antigens.

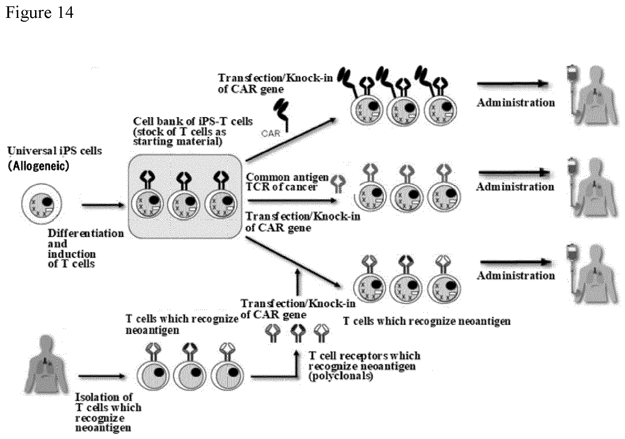

FIG. 14 summarizes the methods shown in FIGS. 11 to 13. The regenerated T cells produced from the same type of universal iPS cells in which peripheral blood T cells have been reprogramed (iPS-T cells) can be used as starting materials for producing T cells transfected with TCR genes or CAR genes. "Universal iPS cells" mean the iPS cells that can be used without causing a rejection even to a patient having any kind of MHC (major histocompatibility complex) due to low immunogenicity. That is, they are the iPS cells which can be administered to a patient without considering MHC matching. Universal iPS cells can be produced by knocking out MHC class I or MHC class II molecules and expressing ligands that inhibit NK cells.

For the transfection of TCR genes or CAR genes, viral vectors used in the production of T cells for conventional replacement therapy or regenerative therapy with T cells may be used. By using iPS-T cells as a starting material, it becomes possible to produce desired T cells as an active ingredient of preventive or therapeutic agent for cancer in a short period of time. In addition, it is also easy to introduce the TCR that recognizes neoantigens, and it is also possible to produce iPS-T cells while maintaining multi-clone properties by transfecting a plurality of TCR genes that recognize different neoantigens.

## Claims

1. A method for producing regenerated T cells via iPS cells, comprising:
(1) a step for preparing cDNAs respectively encoding a TCR α chain and a TCR β chain from individual cells in a CD106-positive T cell population obtained from subjects and having reactivity with a tumor-related antigen;
(2) a step for reprogramming peripheral blood mononuclear cells which are obtained from the subjects and from which B cells and T cells have been removed or T cells into iPS cells, and selecting iPS cell clones having high efficiency of differentiation into T cells from the obtained iPS cells;
(3) a step for introducing the cDNAs into the iPS cell clones, hematopoietic stem cells differentiated from the iPS cell clones, immature T cells differentiated from the hematopoietic stem cells, or mature T cells differentiated from the immature T cells; and
(4) a step for performing the differentiation of the iPS cell clones having the cDNAs introduced therein, the hematopoietic stem cells or the immature T cells which have been obtained in step (3) into mature T cells and the proliferation of the mature T cells.

2. The method according to claim 1, wherein step (2) is carried out prior to or in parallel with step (1).

3. The method according to claim 1 or 2, wherein the subjects in steps (1) and (2) are the same individuals and are those to be prevented or treated of cancer.

4. The method according to claim 1 or 2, wherein the subjects in steps (1) and (2) are separate individuals from each other and the subjects in step (2) are those to be prevented or treated of cancer.

5. The method according to claim 1 or 2, wherein the subjects in steps (1) and (2) are separate individuals from each other and the subjects in step (1) are those to be prevented or treated of cancer.

6. The method according to claim 1 or 2, wherein the subjects in steps (1) and (2) are the same individuals or separate individuals from each other, and the subjects different from the subjects in steps (1) and (2) are those to be prevented or treated of cancer.

7. The method according to claim 1 or 2, further comprising a step for selecting, from the T cells into which the cDNAs obtained in step (4) have been introduced, the T cells which do not exhibit an alloreaction to the cells derived from subjects to be prevented or treated of cancer.

8. The method according to claim 1 or 2, further comprising a step for selecting, from the cDNAs prepared in step (1), the cDNAs encoding a TCR that do not induce an alloreaction to the cells derived from subjects to be prevented or treated of cancer.

9. The method according to claim 7 or 8, wherein the cells derived from subjects to be prevented or treated of cancer are peripheral blood mononuclear cells.

10. The method according to any one of claims 1 to 9, wherein the introduction of the cDNAs into the iPS cell clones, the hematopoietic stem cells differentiated from the iPS cell clones, the immature T cells differentiated from the hematopoietic stem cells, or mature T cells differentiated from the immature T cells in step (3) uses a virus vector, a non-viral vector, or a genome editing technique.

11. The method according to claim 10, wherein the genome editing technique is CRISPR/Cas9 or TALEN.

12. The method according to claim 10, wherein the non-viral vector is a transposon vector.

13. The method according to claim 12, wherein the transposon vector is piggyBac^{®} transposon vector.

14. The method according to any one of claims 1 to 13, wherein the step (4) for performing the differentiation of the iPS cell clones having the cDNAs introduced therein, the hematopoietic stem cells or the immature T cells into mature T cells and the proliferation of the mature T cells is carried out in the presence of feeder cells and PHA (phytohemagglutinin), RetroNectin^{®} and anti-CD3 antibodies, or anti-CD3 antibodies and anti-CD28 antibodies.

15. The method according to claim 14, wherein the feeder cells are autologous or allogeneic peripheral blood mononuclear cells.

16. The method according to any one of claims 1 to 15, wherein the subjects in step (1) or (2) are patients of hepatoma, hepatoblastoma, gastric cancer, esophageal cancer, lung cancer, pancreatic cancer, renal cell cancer, breast cancer, ovarian cancer, skin cancer, such as malignant melanoma, bladder cancer, head and neck cancer, uterine cancer, cervical cancer, glioblastoma, prostate cancer, neuroblast, chronic lymphocytic leukemia, thyroid papilla cancer, colon cancer, brain tumor, sarcoma, or B-cell non-Hodgkin's lymphoma.

17. The method according to any one of claims 1 to 15, wherein the subjects in step (1) or (2) are highly immunogenic cancer patients.

18. The method according to claim 17, wherein the highly immunogenic cancer is hepatoma, hepatoblastoma, colon cancer, lung cancer, or malignant melanoma.

19. The method according to any one of claims 1 to 18, wherein the T cell population in step (1) is CD3/CD106 double-positive.

20. The method according to any one of claims 1 to 19, wherein the hematopoietic stem cells are CD34/CD43 double-positive.

21. The method according to any one of claims 1 to 20, wherein the immature T cells are CD8 α chain/β chain double-positive.

22. The method according to any one of claims 1 to 21, wherein the iPS cell clones with high differentiation efficiency selected in step (2), the hematopoietic stem cells differentiated from the iPS cell clones in step (3), the immature T cells differentiated from the hematopoietic stem cells, or the mature T cells differentiated from the immature T cells are preserved to construct a master cell bank.

23. The method according to claim 22, wherein the preservation is cryopreservation.

24. The method according to claim 22, wherein steps (3) and (4) are performed on the iPS cell clones, the hematopoietic stem cells, the immature T cells, or the mature T cells preserved in the master cell bank.

25. The master cell bank according to claim 22, comprising the iPS cell clones, the hematopoietic stem cells, the immature T cells, or the mature T cells.

26. Regenerated T cells produced by the method according to any one of claims 1 to 24.

27. A pharmaceutical composition, containing the regenerated T cells according to claim 26.

28. A method for preventing or treating cancer using the pharmaceutical composition according to claim 27.
